# EUROPÄISCHE PATENTANMELDUNG

(11) **EP 0 648 479 A1**
(43) Veröffentlichungstag der Anmeldung: **19.04.1995**
(21) Anmeldenummer: 93117108.6
(22) Anmeldetag: 22.10.1993
(51) Int. Cl.: A61F 2/66

(54) **Sprunggelenkfederelement für Beinprothesen und Kunstfuss**

(30) Priorität: 14.10.1993 DE 9315665 U
(71) Anmelder: IPOS GMBH & CO. KG., D-21337 Lüneburg (DE)
(72) Erfinder: Schneider-Nieskens, Reinhold, D-21337 Lüneburg (DE)
(74) Vertreter: Richter, Werdermann & Gerbaulet

(57) **Zusammenfassung**

Um eine Fußbewegung im Sinne einer Pro-Subination um die Fußlängsachse zu erreichen, besteht das Sprungfederelement mit einem Versteifungskörper (10) aus einem U-förmigen Profil (16), dessen jeweilige Schenkel bei Belastung elastisch bewegbar sind, so daß ein Kunstfuß mit einem dem natürlichen Bewegungsablauf entsprechenden Funktionsmechanismus erhalten wird.

## Beschreibung

Die Erfindung betrifft ein Sprunggelenkfederelement für Beinprothesen, insbesondere aus einem Schaumkunststoff-Fußformteil mit einem metallischen Versteifungskörper.

Die Erfindung betrifft ferner einen Kunstfuß für eine Beinprothese aus einem Schaumkunststoff-Fußformteil mit einem aus einem in seinem Sohlenbereich eingebetteten, plattenförmigen metallischen Versteifungskörper, der im Ballenbereich mit einer fußblallengleich verlaufenden Kröpfung zur Unterstützung der Fußabrollfunktion ausgebildet ist, und mit - jeweils als elastisch nachgiebige Abfederung dienend - einem Vorfußkern und einer im Fersenbereich angeordneten Lasche bestehenden Funktionskern, wobei der Funktionskern und der Versteifungskörper fest miteinander verbunden sind und der Versteifungskörper aus einer oder mehreren etwa gleich langen, übereinanderliegenden Blattfedern besteht, die dem Abrollprofil des Fußes entsprechend geformt sind.

Es ist seit langem bekannt, als Kunstfuß-Material Polyurethan-Schaumkunststoff einzusetzen, welches den Vorteil eines niedrigen Gewichtes aufweist. Wie beispielsweise in der US-A-3,335,428 vorgeschlagen, kann das Bein-Prothesen-Fußteil auch aus elastischem Kunststoff unterschiedlicher Härte geformt sein.

Ferner ist aus der DE-C-354 246 ein Kunstfuß bekannt, bei dem im Sohlenbereich des Kunstfußes eine Metallschiene eingebettet ist. Dieser künstliche Fuß soll das Weitausschreiten eines Fuß- oder Beinamputierten ermöglichen, da das Weitausschreiten das schräg nach hinten Verlegen des Unterschenkels, also das Nachgeben der Ferse des mit seiner ganzen Unterfläche auf dem Boden aufruhenden Fußes bedingt. Er soll ferner das leichte Wiederaufrichten des Unterschenkels bedingen, ohne daß dieser die Senkrechtlage überschreitet, während das Verlegen des Körpergewichtes nach vorn und das Anheben der Ferse durch die übliche Nachgiebigkeit des Mittelfuß- und Zehenteils des Kunstfußes ermöglicht werden soll. Diese Nachgiebigkeit soll durch Einlagern einer durch den Sohlen- und Fersenteil hindurchgehenden Federplatte erzielt werden. Um nunmehr die Ausladung des Unterschenkels schräg nach hinten herbeiführen zu können, sieht diese bekannte Ausführungsform eines Kunstfußes eine Verbindung zwischen dem starren Unterschenkel und der Federplatte vor, bei der sich der Schenkel in Art einer Wiege aus seiner Aufrechtstellung nach rückwärts bewegt und bei Verlegung des Körpergewichtes nach vorne wieder in die erste übergeht. Diese Schaukelbewegung erfolgt dadurch, daß der keilförmig zulaufende, starre Unterschenkel in einem Sattel sitzt, dessen eine Schräge vom Spann des Kunstfußes und dessen Gegenschräge von einer Abzweigung der Federplatte gebildet wird. Zur weiteren Abfederung befindet sich zwischen der Abzweigung und der eigentlichen Federplatte ein zusammendrückbares Keilkissen, z.B. aus weichem Gummi. Damit beim Anstoßen der vorderen Keilfläche am Schenkel gegen den Fuß kein unerwünschtes Geräusch auftritt, besteht der Vorder- bzw. Mittelfuß aus einer schmiegsamen oder leich zusammendrückbaren, jedenfalls geräuschdämpfenden Masse, vorzugsweise Filz. Mit einer Kappe aus diesem Stoff ist auch die Widerlagerfläche des Unterschenkels überzogen. Die Abzweigung der Federplatte ist bei diesem künstlichen Fuß mit der hinteren Keilfläche des Unterschenkels fest verbunden. Dabei wird jedoch die Abzweigung nicht scharf abgebogen, da eine wiegende oder schaukelnde Bewegung angestrebt wird. Sie ist daher mit ihrem Knie gleitend auf der Platte angebracht. Bei diesem bekannten Kunstfuß ist somit eine Stahlfeder mit federnden Eigenschaften eingesetzt, die bis in den Vorfuß reicht. Die wichtigen Elastizitätsunterschiede zwischen Mittelfuß und dem Vorfuß sind hier jedoch konstruktiv nicht berücksichtigt. Die verwendete Stahlfeder ermöglicht jedoch kein natürliches Abrollen; auch sind die Verbindungsprobleme zwischen den verschiedenen Materialien im elastischen Bereich nicht konstruktiv gelöst.

Aus der DE-C-361 972 ist ferner ein künstlicher Fuß mit einer aus mehreren, gegeneinander abgestuften Blattfedern bestehenden Längsfederung bekannt, deren Blattfedern mit ihren rückwärtigen Enden auf der unteren Seite eines den hinteren Fußteil bildenden, aber von der Sohle durch eine Zwischenschicht getrennten starren Klotzes befestigt sind, während die vorderen, in geeigneter doppelter Krümmung abwärtsgeführten Federenden unmittelbar auf die zweckmäßig durch ein Schutzblech bedeckte Sohle aufdrücken.

Bei einem aus der US-A-2 556 525 bekannten Kunstfuß für Beinprothesen ist in einem äußeren Schaumkunststoff-Fußformteil ein steifer, jedoch flexibler Kunststoffteil eingelassen, der sich über die gesamte Länge des Fußes erstreckt, wobei in diesem steifen, jedoch flexiblen Kunststoffteil eine Metalleinlage aus Federstahl eingebettet ist. Auch wenn ein innerer, teilweise flexibler Kunststoffteil mit einer Metalleinlage aus Federstahl bei diesem bekannten Kunstfuß verwendet wird, so ist es bei diesem Kunstfuß nicht möglich, die Abrollfunktion auf die von der Natur vorgegebene Rüttelungslinie zu legen. Trotz der Verwendung eines flexiblen Kunststoffteils und einer Metalleinlage aus Federstahl ist eine ausreichende Beweglichkeit im Zehengrundgelenk, wie dies in der Natur gegeben ist, nicht möglich.

In der DE-A-22 37 758 wird ein allseits bewegliches Fußgelenk für Rohrskelettprothesen vorgeschlagen, das auf einer in einem Trägerelement verschraubten Achse ein Gelenklager besitzt, auf welchem ein Augenbolzen aufgepreßt ist, der im Preßsitz auf einem handelsüblichen Gelenklager den schwenkbaren Augenbolzen aufnimmt, dessen Schaft im Trägerelement gleitend gelenkig in einem Gelenklager geführt am Ende eine Abschlußplatte trägt, die, vorn und hinten mit elastischen Pufferelementen beaufschlagt, die Bewegungsbegrenzung steuert.

Um einen Kunstfuß für Beinprothesen mit einem über einen längeren Zeitraum vorgegebenen Abrollwiderstand hoher Elastizität und einer Einknickmöglichkeit im Zehengrundgelenk zu schaffen, ist in der DE-C-23 41 887 ein Kunstfuß vorgeschlagen worden, bei dem die Vorfußelastizität durch Einsatz eines homogenen Elastomers gewährleistet wird, d.h. es wird im Vorfuß eine Rückstellelastizität berücksichtigt, die weit über bis dahin geformte Polstereffekte hinausging. Es ist auch schon vorgeschlagen worden, einen Versteifungskörper aus zwei etwa gleich langen, übereinanderliegenden Blattfedern zu verwenden, die dem Abrollprofil des Fußes entsprechend geformt sind.

Es ist Aufgabe der vorliegenden Erfindung, das eingangs genannte Sprungfederelement derart weiterzuentwickeln, daß es eine Fußbewegung im Sinne einer Pro-Subination um die Fußlängsachse gestattet. Ferner ist es Aufgabe der vorliegenden Erfindung, einen Kunstfuß der bekannten Art weiterzuentwickeln, daß ein dem natürlichen Bewegungsablauf entsprechender Funktionsmechanismus geschaffen wird.

Diese Aufgabe wird zum einen durch das im Anspruch 1 beschriebene Sprunggelenkfederelement gelöst, das ein U-förmiges Profil aufweist, dessen jeweilige Schenkel bei Belastung elastisch aufeinanderzu bewegbar sind. Der Vorteil dieser Ausführungsform besteht darin, daß ein materialsparendes U-Profil verwendet werden kann und so die Herstellkosten auf ein Minimum herabgesetzt werden.

Weiterbildungen des Sprunggelenkfederelementes sind in den Ansprüchen 2 bis 9 beschrieben. So ist zur Erhöhung der Elastizität vorgesehen, daß die auslaufenden Enden des U-Profils, vorzugsweise mit Ausnahme der zur Befestigung an einer Aufnahme eines Beinanschlußteiles benötigten Teilbereiche, sich zum freien Ende hin in der Dicke (Materialstärke) verjüngen. Vorzugsweise sind die Schenkelenden verdickt ausgeführt, in einer speziellen Ausführungsform an den Innenseite mit Verdickungen ausgestattet, die sich über die gesamte Breite ausbilden. Diese Verdickungen liefern hinsichtlich einer Befestigung, z.B. mittels eines Bolzens, eine ausreichende Halterung bzw. Führung, ohne daß die Gefahr von Materialbrüchen oder in elastischen Verformungen besteht.

Nach einer weiteren Ausführungsform sind die Schenkelenden jeweils mit mindestens einer, vorzugsweise zwei nebeneinanderliegenden Bohrungen versehen. Durch die übereinanderliegenden Bohrungen verschiedener Schenkel ist jeweils ein Bolzen steckbar.

Eine weitere Verbesserung hinsichtlich der Pro-Subination um die Fußlängsachse bei gleichzeitiger Ermöglichung einer Rotation um die Beinlängsachse ergibt sich, wenn das U-förmige Profil zumindest über einen Teil seiner Längsachse gehende Aussparungen erhält. Vorzugsweise bestehen diese Aussparungen aus Längsschlitzen, die sich in einer weiteren Verbesserung vom ersten Schenkel über die Basis bis zum zweiten Schenkel des U-förmigen Profils durchgehend erstrecken.

Eine ausreichende Stützung bei gleichzeitiger Schaffung einer hinreichenden Elastizität wird erreicht, wenn die Breite des U-Profils etwa 0,4 bis 0,6 mal der Länge beträgt. Vorzugsweise ist das U-Profil in einer Draufsicht betrachtet im wesentlichen rechteckig. Verwendet man als Material für das U-Profil eine Titan-Aluminium-Legierung, so kann das Sprunggelenkfederelement mit wenig Gewicht ausgebildet werden. Das vorbeschriebene Sprunggelenkfederelement wird bevorzugt in einen Kunstfuß der eingangs genannten Art eingebaut, wo es als Verbindung zwischen einem Versteifungskörper und der Aufnahme eines Beinanschlußteils dient, das mit einem Schenkel des U-Profils verbunden ist. Die im Kunstfuß verwendeten Blattfedern bzw. die Blattfeder besteht aus Kohlefaser oder Titan oder einer Titanlegierung.

Ausführungsbeispiele der Erfindung sind in den Zeichnungen dargestellt. Es zeigen
Fig. 1 eine perspektivische Ansicht eines Sprunggelenkfederelementes und
Fig. 2 einen Querschnitt durch einen Kunstfuß.

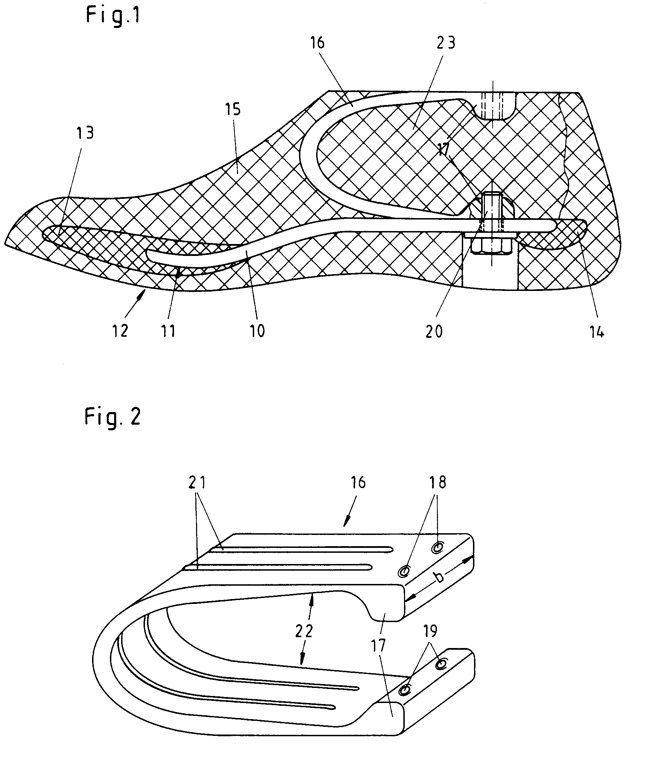

Der in Fig. 1 dargestellte Versteifungskörper 10 besitzt eine nach unten gerichtete Kröpfung 11 im Ballenbereich 12 des Kunstfußes, die dem Abrollprofil des Fußes entsprechend geformt ist. Der Versteifungskörper 10 ist in einem Vorfußkern 13 ebenso eingebettet wie in einer im Fersenbereich liegenden Lasche 13. Der aus den Teilen 10,13 und 14 bestehende Funktionskern ist von einem Schaumkunststoff-Fußformteil 15 umschäumt, das im rückwärtigen Teil einen Hohlraum zur Aufnahme von Gelenken oder Befestigungselementen besitzt. Die nach unten gerichtete Kröpfung 11 ist dergestalt ausgebildet, daß die Abrollfunktion auf die von der Natur vorgegebene vordere Drittelungslinie (Zehengrundgelenklinie) gelegt ist. Dabei ist an der Kröpfung 11 des Versteifungskörpers 10 ein aus einem Elastomer bestehender Vorfußkern 13 anvulkanisiert, der in seiner unteren Abschlußlinie der äußeren Form des Fußballens entspricht. Der Vorfußkern 13 ist durch die umschäumte Struktur völlig alterungsbeständig, so daß der Abrollwiderstand über längere Zeit unveränderlich vorbestimmbar ist. In entsprechender Weise ist im Fersenteil die Lasche 14 anvulkanisiert.

Der als Blattfeder ausgebildete Versteifungskörper 10 ist an seinem hinteren Ende mit dem erfindungsgemäßen Sprunggelenkfederelement 16 in Form eines U-Profils verbunden. Die Basis des U-Profils weist in Richtung der Fußspitze bzw. die freien Schenkelenden des U-Profils zur Ferse. Das U-Profil, das in Fig.2 näher dargestellt ist, besitzt an seinen Enden Verdickungen 17, die sich über die gesamte Breite b derstrecken. In diesen Verdickungen 17 befinden sich nebeneinanderliegende Bohrungen 18 bzw. 19 , die so übereinanderliegend angeordnet sind, daß der Fig.1 entnehmbare Bolzen 20 durch jeweils übereinanderliegende Bohrungen der beiden Schenkel steckbar ist. Dieser Bolzen 20 ist mit einer Aufnahme eines Beinanschlußteils verschraubt oder sonst wie fest verbunden. Das U-Profil 16 besitzt ferner bis an den Verdickungsbereich 17 reichende Schlitze 21, die sich durchgehend von dem einen freien Schenkel über die Basis bis zum Ende des anderen freien Schenkels erstrecken. Diese Schlitze, hier zwei Schlitze 21, sind parallel zueinander und parallel zur nicht dargestellten Längsachse geführt. Das U-Profil ist so ausgestaltet, daß möglichst beider Schenkel, zumindest jedoch ein Schenkel, zum freien Ende eine Dickenabnahme 22 zeigt, wodurch die Elastizität des freien Schenkels in dem Bereich erhöht wird. Wie Fig. 1 zu entnehmen , ist zwischen den freien Schenkeln des U-Profils 16 ein weicher Polyurethanschaum 23 eingesetzt. Die Blattfeder als Verstärkungselement 10 ist aus Kohlefaser oder Titan gefertigt.

Der dargestellte Kunstfuß besitzt den Vorteil, daß er weiche, ineinanderlaufende Bewegungen ermöglicht. Für verschiedenartige Fußgrößen können jeweils verschiedene U-Federsysteme verwendet werden, wobei die Dimensionierung der U-Profile einen präzise auf die Erfordernisse der Bewegung abgestimmten Widerstand erlaubt. Gezielte Flexibilitäten in die Konstruktion werden darüber hinaus durch die Längsschlitze 21 eingegeben.

## Patentansprüche

1. Sprunggelenkfederelement für Beinprothesen, insbesondere aus einem Schaumkunststoff-Fußformteil mit einem metallischen Versteifungskörper (10), gekennzeichnet durch ein U-förmiges Profil (16), dessen jeweilige Schenkel bei Belastung elastisch aufeinanderzu bewegbar sind.

2. Sprunggelenkfederelement nach Anspruch 1, dadurch gekennzeichnet, daß die auslaufenden Enden, vorzugsweise mit Ausnahme der zur Befestigung an einer Aufnahme eines Beinanschlußteiles benötigten Teilbereiche, sich zum freien Ende hin in der Dicke (Materialstärke) verjüngen.

3. Sprunggelenkfederelement nach Anspruch 1 oder 2, dadurch gekennzeichnet, daß die Schenkelenden Verdickungen (17) aufweisen, die vorzugsweise an den Innenseiten über die gesamte Breite (b) ausgebildet sind.

4. Sprunggelenkfederelement nach einem der Ansprüche 1 bis 3, dadurch gekennzeichnet, daß die Schenkelenden jeweils mindestens eine, vorzugsweise zwei nebeneinanderliegende Bohrungen (18,19) zum Durchstecken jeweils eines einzigen Bolzens (20) durch zwei übereinanderliegende Bohrungen aufweisen.

5. Sprunggelenkfederelement nach einem der Ansprüche 1 bis 4, dadurch gekennzeichnet, daß das U-förmige Profil (16) zumindest über einen Teil seiner Längsachse gehende Aussparungen (21) aufweist.

6. Sprunggelenkfederelement nach Anspruch 5, dadurch gekennzeichnet, daß die Aussparungen Längsschlitze (21) sind, die sich vorzugsweise vom ersten Schenkel bis zum zweiten Schenkel durchgehend erstrecken.

7. Sprunggelenkfederelement nach einem der Ansprüche 1 bis 6, dadurch gekennzeichnet, daß die Breite (b) des U-Profils (16) etwa 0,4 bis 0,6 mal der Länge beträgt.

8. Sprunggelenkfederelement nach einem der Ansprüche 1 bis 7, dadurch gekennzeichnet, daß das U-Profil (16) in einer Draufsicht betrachtet im wesentlichen rechteckig ist.

9. Sprunggelenkfederelement nach einem der Ansprüche 1 bis 8, dadurch gekennzeichnet, daß das U-Profil aus einer Ti-Al-Legierung besteht.

10. Kunstfuß für eine Beinprothese aus einem Schaumkunststoff-Fußformteil mit einem aus einem in seinem Sohlenbereich eingebetteten plattenförmigen metallischen Versteifungskörper, der im Ballenbereich mit einer fußballengleich verlaufenden Kröpfung zur Unterstützung der Fußabrollfunktion ausgebildet ist, und mit - jeweils als elastisch nachgiebige Abfederung dienend - einem Vorfußkern und einer im Fersenbereich angeordneten Lasche bestehenden Funktonskern, wobei der Funktionskern und der Versteifungskörper fest miteinander verbunden sind und der Versteifungskörper aus einer oder mehreren etwa gleich langen übereinanderliegenden Blattferder(n) besteht, die dem Abrollprofil des Fußes entsprechend geformt sind, dadurch gekennzeichnet, daß der Versteifungskörper mit einem Schenkel eines bei Bebelastung elastisch federnden U-Profils und der andere Schenkel mit der Aufnahme eines Beinanschlußteils verbunden ist.

11. Kunstfuß nach Anspruch 10, dadurch gekennzeichnet, daß die Blattfeder(n) aus Kohlefaser oder Titan oder einer Titanlegierung besteht/bestehen.

12. Kunstfuß nach Anspruch 10 oder 11, dadurch gekennzeichnet, daß der Zwischenraum zwischen dem freien Schenkel des U-Profils (16) mit einem weichen Polyurethanschaum (23) ausgefüllt ist.
